# EUROPEAN PATENT SPECIFICATION

(11) **EP 3 229 605 B1**
(45) Date of publication and mention of the grant of the patent: **02.10.2019**
(21) Application number: 15866796.4
(22) Date of filing: 07.12.2015
(51) Int. Cl.: A23K 20/158, A23K 20/195, A23K 50/75

(54) **COMPOSITION INHIBITING GRAM-NEGATIVE PATHOGENS IN GALLOANSERANS**
ZUSAMMENSETZUNG ZUR HEMMUNG VON GRAM-NEGATIVEN PATHOGENEN IN GALLOANSERANEN
COMPOSITION INHIBANT LES PATHOGÈNES À GRAM-NÉGATIF CHEZ LE GALLOANSERANS

(30) Priority: 09.12.2014 SE 1400579
(43) Date of publication of application: 18.10.2017
(73) Proprietor: Perstorp AB, 284 80 Perstorp (SE)
(72) Inventor: VAN IMMERSEEL, Filip, 9810 Eke (BE); Van DRIESSCHE, Karolien, 599240 Zele (BE); DUCATELLE, Richard, 9790 Wortegem-Petegemm (BE); SCHWARZER, Conrad Gerard, 3583 Beringen (BE)
(74) Representative: Hoffmann Eitle
(86) International application number: PCT/SE2015/000080
(87) International publication number: WO 2016/093757

(56) References cited:
- EP-A1- 1 059 041
- EP-A1- 1 059 041
- EP-A1- 1 224 870
- EP-A1- 2 215 913
- EP-A1- 2 215 913
- EP-A1- 2 294 924
- WO-A1-99/66804
- WO-A1-2006/085346
- WO-A2-2010/106488
- WO-A2-2011/002298
- CN-A- 102 204 633
- JP-A- 2010 215 542
- US-A1- 2004 116 523
- US-A1- 2004 116 523

## Description

### FIELD OF THE INVENTION

The present invention refers to a glycerol ester composition of at least one short chain fatty acid for use in inhibiting a bacterium of the genus *Salmonella* in the gastric tract of galloanserans. The glycerol ester composition comprises at least 75% by weight of glyceryl trivalerate, below 25% by weight of glyceryl divalerate and below 8% by weight of glyceryl monovalerate.

### BACKGROUND OF THE INVENTION

Foodborne illness (also foodborne disease or food poisoning) is any illness resulting from the consumption of contaminated food, pathogenic bacteria, viruses, or parasites that contaminate food as well as chemical or natural toxins such as poisonous mushrooms. Symptoms often include vomiting, fever, and aches, and may include diarrhea. Foodbome illness usually arises from improper handling, preparation or food storage; factors that the consumer to some extent can control himself. However, in order to minimize the number of cases of foodborne illness, it is vital that the food that is produced and sold to consumers is safe.

Among the most common bacteria causative of foodborne illness in human are *Campylobacter jejuni, Salmonella* and *Eschericia coli.* These three bacteria are all classified as gram-negative. Gram staining differentiates bacteria by the chemical and physical properties of their cell walls by detecting peptidoglycan, which is present in a thick layer in gram-positive bacteria. In a Gram stain test, gram-positive bacteria retain the crystal violet dye, while a counterstain (commonly safranin or fuchsine) added after the crystal violet gives all gram-negative bacteria a red or pink coloring.

*Salmonella* is consistently the most common bacterial pathogen in laboratory confirmed foodborne illness cases. There are two distinct species of the genus *Salmonella: Salmonella enterica* and *Salmonella bongori. S. enterica* is found primarily in poultry and poultry products. In many cases *Salmonella* is freely living in the animals during their growth, without the chickens showing any symptoms of illness. The result is that *Salmonella* is still present in the poultry meat and the eggs, capable of infecting humans when these products are consumed. To protect against *Salmonella* infection, heating food for at least ten minutes at 75 °C is recommended, so that the center of the food reaches this temperature. Incompletely heated food or contamination between different foods during preparation and/or cooking can result in consumers getting infected with *Salmonella.*

Most people infected with *Salmonella* develop diarrhea, fever, vomiting, and abdominal cramps 12 to 72 hours after infection. In most cases, the illness lasts four to seven days, and the majority of infected people recover without treatment. In some cases, the diarrhea may be so severe that the patient becomes dangerously dehydrated and must be hospitalized. The elderly, infants, and those with impaired immune systems are more likely to develop severe illness. In severe cases, the *Salmonella* infection may spread from the intestines to the blood stream, and then to other body sites and can cause death, unless the person is treated promptly with antibiotics. *Salmonella* is estimated to cause more than 1.2 million illnesses each year in the United States, with more than 23,000 hospitalizations and 450 deaths.

Because of the risk of consumers getting infected with *Salmonella,* it is of great interest to farmers, the food industry and end consumers to find ways to control *Salmonella* and other pathogens in the animals that we consume, for example in poultry. This has earlier been accomplished by routinely adding antibiotics to the feed. Since the ban of growth promoting antibiotics in the European Union in 2006, there is a great need for alternative methods to prevent *Salmonella* infections in animals and especially in galloanserae. Galloanserae are divided into the subgroups galliformes (landfowls) like chickens, turkeys, grouses and pheasants, and anseriforms (waterfowls) like ducks, geese and swans.

The use of different short chain fatty acids as antibacterial agents against for example *Salmonella* is known since a long time in the prior art, for instance in the documents EP 2 215 913 A1 which describes the use of valeric acid in salmonella suppression in poultry and WO99/66804 with a more general teaching. Using glycerol esters of short chain fatty acids is an alternative way to distribute these acids to the gastrointestinal tract of an animal. There are several advantages associated with distributing the acids in the form of glycerol esters. These acids are often corrosive and have a pungent, unpleasant odor, which causes handling problems.

The glycerol esters do not have these problems with smell or corrosiveness. Distributing short chain fatty acids to an animal in the form of glycerol esters is also a good way to transport the fatty acids to the lower regions of the gastrointestinal tract of an animal. Free short chain fatty acids are likely to be adsorbed in the higher regions of the gastrointestinal canal and will never reach the cecum or large intestine. Glycerol esters on the other hand will probably to a large extent reach these regions in a partly dissociated form and a large proportion of the fatty acid will be released and adsorbed in these regions.

The present invention shows that a specific triester of glycerol is more effective than other comparable esters for inhibiting growth of *Salmonella* in chickens. A glycerol ester composition, comprising mainly glycerol trivalerate, has surprisingly been found to reduce bacterial count in the ceaca of broiler chicken infected with *Salmonella,* to a greater extent compared to a glycerol ester composition comprising mainly glycerol tripropionate or glycerol tributyrate. The bacterial count of *Salmonella* in the chickens fed glycerol trivalerate was found to be lowered also compared to that in chickens fed glycerol monovalerate. This shows that a glycerol ester composition according to the present invention, comprising mainly glycerol trivalerate, is a particularly good choice for inhibiting gram-negative bacteria like *Salmonella* in galloanserans.

### DETAILED DESCRIPTION OF THE INVENTION

The present invention refers to the use of a glycerol ester composition comprising at least one short chain fatty acid, for controlling a bacterium of the genus *Salmonella* in the gastric tract of galloanserans, such as broiler chicken. The glycerol ester composition comprises at least 75% by weight of glyceryl trivalerate, below 25% by weight of glyceryl divalerate and below 8% by weight of glyceryl monovalerate. The present invention is accordingly directed to said glycerol ester composition for use in inhibiting a bacterium of the genus *Salmonella* in the gastric tract of galloanserans. The bacterium of the genus *Salmonella* can be such as *Salmonella enterica.*

To prevent galloanserae, and later on in the value chain humans, from getting infected with common foodborne pathogens like the gram-negative bacteria *Salmonella,* the use of different short chain fatty acids has earlier been described.

The present invention shows that using a glycerol ester composition comprising mainly glycerol trivalerate is a more efficient way to control a bacterium of the genus Salmonella in the gastric tract of galloanserans than using either a composition comprising mainly glycerol tripropionate, a composition comprising mainly glycerol tributyrate or a composition comprising mainly glycerol monovalerate.

According to one embodiment of the present invention the glycerol ester composition comprises at least 80% by weight of glyceryl trivalerate, below 20% by weight of glyceryl divalerate and below 5% by weight of glyceryl monovalerate.

According to a preferred embodiment of the present invention the glycerol ester composition comprises at least 85% by weight of glyceryl trivalerate, below 15% by weight of glyceryl divalerate and below 4% by weight of glyceryl monovalerate.

According to a more preferred embodiment of the present invention the glycerol ester composition comprises at least 90% by weight of glyceryl trivalerate, below 10% by weight of glyceryl divalerate and below 2.5% by weight of glyceryl monovalerate.

Free valeric acid has an unpleasant smell, which causes handling problems. These problems can be avoided by distributing the valeric acid in the form of glycerol esters. According to one embodiment of the present invention, the amount of free valeric acid in the glycerol ester composition is below 1% and preferably below 0.5% by weight. Keeping down the amount of free valeric acid also ensures that the pH in the glycerol ester composition is kept at a level where the glycerol ester will not undergo hydrolyzation into glycerol and free acid, hence, the product is kept stable.

According to one embodiment of the present invention the glycerol ester composition is adsorbed on an inert carrier, such as a silica carrier. This allows the composition to be distributed as a dry product. Such a silica carrier preferably comprises porous silica particles with an average particle size of 20-70 µm. According to one embodiment of the present invention, the glycerol ester composition is adsorbed on silica particles in a weight ratio of 50-80 % glycerol ester and 20-50 % silica particles.

The glycerol ester composition according to the present invention can be added to any commercially available feedstuffs for galloanserans. The glycerol ester composition may be incorporated directly into commercially available feeds or fed supplementary to commercially available feeds.

According to one embodiment of the present invention the amount of glycerol ester composition fed to the galloanserans is from 0.05 to 1.5% by weight and preferably from 0.1 to 1.0% by weight of the galloanserans' daily feed ration.

### EMBODIMENT EXAMPLE

*In vivo* trial showing the efficacy of different glycerol ester compositions to inhibit bacterial infection with *Salmonella enteritis* in the caeca of broiler chicken.

Five groups of 20 chickens in each were fed a commercial feed from day 1 until day 14. The tested compositions were added to the feed at the concentrations below during the whole trial.
Group 1: positive control (no glycerol ester added)
Group 2: 3 kg/ton Glyceryl tripropionate (adsorbed on a silica carrier)
Group 3: 3 kg/ton Glyceryl tributyrate (adsorbed on a silica carrier)
Group 4: 3 kg/ton Glyceryl trivalerate (adsorbed on a silica carrier)
Group 5: 5,6 kg/ton Glyceryl monovalerate (adsorbed on a silica carrier)

On day 1 and 10 swabs were taken to make sure the animals were negative for *S*. *enteritis.* The chickens were inoculated with 2^{∗}10^5 CFU from a wild type *Salmonella Enteritis* on day 10 and swabbed daily until day 14. On day 14 all chickens were euthanized by intravenous soda pentobarbital injection.

The bacterial count of *Salmonella Enteritis* in caeca was determined in log CFU/g. The animals were classified into six different classes according to Table 1 below.

**Table 1.**

| Class | Bacterial count (CFU/g) | Animal infection score |
|---|---|---|
| 1 | < log 0 | 1 |
| 2 | < log 2 | 2 |
| 3 | < log 4 | 3 |
| 4 | < log 6 | 4 |
| 5 | < log 8 | 5 |
| 6 | > log 8 | 6 |

All animals were classified as belonging to one of the six classes above. An animal belonging to class 1, for example, had a bacterial count of less than log 0 (no detection of bacteria) whereas an animal classified as a class 6 had a bacterial count of log 8 or higher. To visualize the results, all animals were assigned a score 1-6, a higher score representing a higher bacterial count (see Table 1). The total infection scores for all 20 animals in each of the five groups of animals tested are shown in Table 2 below.

**Table 2.**

| **Animal group** | **# score 1** | **# score 2** | **# score 3** | **# score 4** | **# score 5** | **# score 6** | **Total infection score for the group** |
|---|---|---|---|---|---|---|---|
| **Control** | 3 | 0 | 9 | 3 | 1 | 4 | 3^{∗}1+0^{∗}2+9^{∗}3+3^{∗}4+1^{∗}5+4^{∗}6 = **71** |
| **Glyceryl tripropionate** | 6 | 2 | 7 | 5 | 0 | 0 | **51** |
| **Glyceryl tributyrate** | 2 | 0 | 9 | 6 | 3 | 0 | **68** |
| **Glyceryl trivalerate** | 8 | 3 | 7 | 1 | 1 | 0 | **44** |
| **Glyceryl monovalerate** | 3 | 0 | 11 | 5 | 1 | 0 | **61** |

The results in Table 2 are visualized in Figure 1 below:

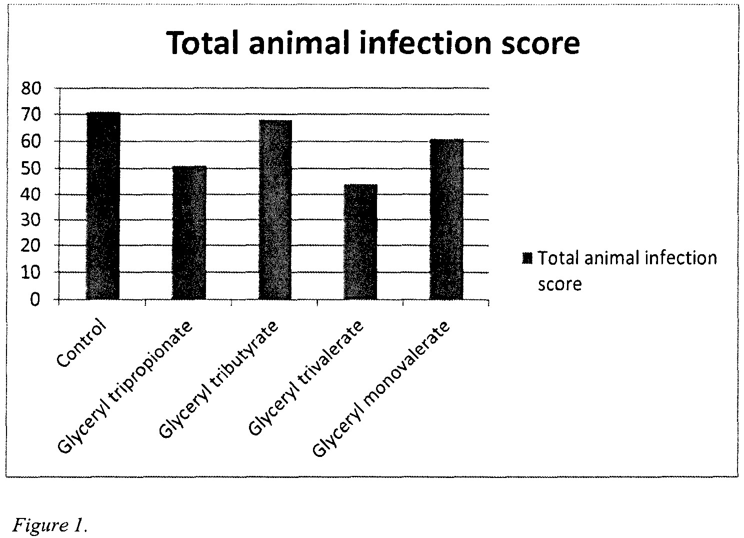

Figure 1 shows that the total animal infection score was lower in the group of chickens that were fed glyceryl trivalerate, compared to the groups where the chickens were fed glyceryl tripropionate, glyceryl tributyrate or glyceryl monovalerate.

The results show that using a glycerol ester composition according to the present invention, comprising a large proportion of glyceryl trivalerate, is an effective way of inhibiting infection with *Salmonella enterica* in galloanserans.

The embodiment example is to be construed as illustrative and not limiting in any way. The glycerol ester composition may for example comprise a blend of glycerol esters of different short chain fatty acids, such as valeric acid, propionic acid and butyric acid. The dosage levels will then have to be calculated with respect to the total amount of glycerol esters.

## Claims

1. A glycerol ester composition of at least one short chain fatty acid, wherein the glycerol ester composition comprises at least 75% by weight of glyceryl trivalerate, below 25% by weight of glyceryl divalerate and below 8% by weight of glyceryl monovalerate, for use in inhibiting gram-negative pathogens in the gastric tract of galloanserans, wherein said gram-negative pathogen is a bacterium of the genus *Salmonella.*

2. A glycerol ester composition for use according to claim 1, wherein said gram-negative pathogen is *Salmonella enterica.*

3. A glycerol ester composition for use according to claim 1 or 2, wherein said galloanserans is a galliform, such as chicken, turkey, grouse or pheasant.

4. A glycerol ester composition for use according to claim 1 or 2, wherein said galloanserans is an anseriform, such as duck, goose or swan.

5. A glycerol ester composition for use according to any of the claims 1-4, wherein said glycerol ester composition comprises at least 80% by weight of glyceryl trivalerate, below 20% by weight of glyceryl divalerate and below 5% by weight of glyceryl monovalerate.

6. A glycerol ester composition for use according to any of the claims 1-4, wherein said glycerol ester composition comprises at least 85% by weight of glyceryl trivalerate, below 15% by weight of glyceryl divalerate and below 4% by weight of glyceryl monovalerate.

7. A glycerol ester composition for use according to any of the claims 1-4 wherein said glycerol ester composition comprises at least 90% by weight of glyceryl trivalerate, below 10% by weight of glyceryl divalerate and below 2.5% by weight of glyceryl monovalerate.

8. A glycerol ester composition for use according to any of the claims 1-7, wherein the amount of free valeric acid in said glycerol ester composition is below 1% by weight.

9. A glycerol ester composition for use according to any of the claims 1-7, wherein the amount of free valeric acid in said glycerol ester composition is below 0.5% by weight.

10. A glycerol ester composition for use according to any of the claims 1-9, wherein said glycerol ester composition is adsorbed on a carrier, such as a silica carrier, thereby allowing said glycerol ester composition to be distributed as a dry product.

11. A glycerol ester composition for use according to claim 10, wherein said carrier comprises porous silica particles with an average particle size of 20-70 µm.

12. A glycerol ester composition for use according to claim 10, wherein said glycerol ester composition is adsorbed on silica particles in a weight ratio of 50-80 % glycerol ester and 20-50 % silica particles.

13. A glycerol ester composition for use according to any of the claims 1-12, wherein said glycerol ester composition is fed to said galloanserans in an amount between 0.05 to 1.5 % by weight of the galloanserans' daily feed ration.

14. A glycerol ester composition for use according to any of the claims 1-12, wherein said glycerol ester composition is fed to said galloanserans in an amount between 0.1 to 1.0 % by weight of the galloanserans' daily feed ration.

## Patentansprüche

1. Glycerinesterzusammensetzung aus mindestens einer kurzkettigen Fettsäure, worin die Glycerinesterzusammensetzung mindestens 75 Gew.-% Glycerintrivalerat, weniger als 25 Gew.-% Glycerindivalerat und weniger als 8 Gew.-% Glycerinmonovalerat umfasst, zur Verwendung bei der Hemmung von gramnegativen Pathogenen im Magentrakt von Galloanserans, worin das gramnegative Pathogen ein Bakterium der Gattung Salmonella ist.

2. Glycerinesterzusammensetzung zur Verwendung gemäß Anspruch 1, worin das gramnegative Pathogen Salmonella enterica ist.

3. Glycerinesterzusammensetzung zur Verwendung gemäß Anspruch 1 oder 2, worin das Galloanserans ein Galliformes wie Huhn, Truthahn, Auerhahn oder Fasan ist.

4. Glycerinesterzusammensetzung zur Verwendung gemäß Anspruch 1 oder 2, wobei das Galloanserans ein Anseriformes wie Ente, Gans oder Schwan ist.

5. Glycerinesterzusammensetzung zur Verwendung gemäß mindestens einem der Ansprüche 1-4, worin die Glycerinesterzusammensetzung mindestens 80 Gew.-% Glycerintrivalerat, weniger als 20 Gew.-% Glycerindivalerat und weniger als 5 Gew.-% Glycerinmonovalerat umfasst.

6. Glycerinesterzusammensetzung zur Verwendung gemäß mindestens einem der Ansprüche 1-4, worin die Glycerinesterzusammensetzung mindestens 85 Gew.-% Glycerintrivalerat, weniger als 15 Gew.-% Glycerindivalerat und weniger als 4 Gew.-% Glycerinmonovalerat umfasst.

7. Glycerinesterzusammensetzung zur Verwendung gemäß mindestens einem der Ansprüche 1-4, worin die Glycerinesterzusammensetzung mindestens 90 Gew.-% Glycerintrivalerat, weniger als 10 Gew.-% Glycerindivalerat und weniger als 2,5 Gew.-% Glycerinmonovalerat umfasst.

8. Glycerinesterzusammensetzung zur Verwendung gemäß mindestens einem der Ansprüche 1-7, worin die Menge an freier Valeriansäure in der Glycerinesterzusammensetzung unter 1 Gew.-% liegt.

9. Glycerinesterzusammensetzung zur Verwendung gemäß mindestens einem der Ansprüche 1-7, worin die Menge an freier Valeriansäure in der Glycerinesterzusammensetzung unter 0,5 Gew.-% liegt.

10. Glycerinesterzusammensetzung zur Verwendung gemäß mindestens einem der Ansprüche 1-9, worin die Glycerinesterzusammensetzung auf einem Träger wie einem Silicaträger adsorbiert ist, wodurch die Verteilung der Glycerinesterzusammensetzung als trockenes Produkt ermöglicht wird.

11. Glycerinesterzusammensetzung zur Verwendung gemäß Anspruch 10, worin der Träger poröse Silicateilchen mit einer durchschnittlichen Teilchengröße von 20-70 µm umfasst.

12. Glycerinesterzusammensetzung zur Verwendung gemäß Anspruch 10, worin die Glycerinesterzusammensetzung auf Silicateilchen in einem Gewichtsverhältnis von 50-80% Glycerinester und 20-50% Silicateilchen adsorbiert ist.

13. Glycerinesterzusammensetzung zur Verwendung gemäß mindestens einem der Ansprüche 1-12, worin die Glycerinesterzusammensetzung den Galloanserans in einer Menge zwischen 0,05 bis 1,5 Gew.-% der täglichen Futterration der Galloanserans gefüttert wird.

14. Glycerinesterzusammensetzung zur Verwendung gemäß mindestens einem der Ansprüche 1-12, worin die Glycerinesterzusammensetzung den Galloanserans in einer Menge zwischen 0,1 bis 1,0 Gew.-% der täglichen Futterration der Galloanserans gefüttert wird.

## Revendications

1. Composition d'ester de glycérol d'au moins un acide gras à chaîne courte, dans laquelle la composition d'ester de glycérol comprend au moins 75 % en poids de trivalérate de glycéryle, moins de 25 % en poids de divalérate de glycéryle et moins de 8 % en poids de monovalérate de glycéryle, pour utilisation afin d'inhiber des agents pathogènes à Gram négatif dans le tractus gastrique de galloanserans, dans laquelle ledit agent pathogène à Gram négatif est une bactérie du genre *Salmonella.*

2. Composition d'ester de glycérol à utiliser selon la revendication 1, dans laquelle ledit agent pathogène à Gram négatif est *Salmonella enterica.*

3. Composition d'ester de glycérol à utiliser selon la revendication 1 ou 2, dans laquelle ledit galloanserans est un galliforme, tel que le poulet, la dinde, le coq de bruyère ou le faisan.

4. Composition d'ester de glycérol à utiliser selon la revendication 1 ou 2, dans laquelle ledit galloanserans est un ansériforme, tel qu'un canard, une oie ou un cygne.

5. Composition d'ester de glycérol à utiliser selon l'une quelconque des revendications 1 à 4, dans laquelle ladite composition d'ester de glycérol comprend au moins 80 % en poids de trivalérate de glycéryle, moins de 20 % en poids de divalérate de glycéryle et moins de 5 % en poids de monovalérate de glycéryle.

6. Composition d'ester de glycérol à utiliser selon l'une quelconque des revendications 1 à 4, dans laquelle ladite composition d'ester de glycérol comprend au moins 85 % en poids de trivalérate de glycéryle, moins de 15 % en poids de divalérate de glycéryle et moins de 4 % en poids de monovalérate de glycéryle.

7. Composition d'ester de glycérol à utiliser selon l'une quelconque des revendications 1 à 4, dans laquelle ladite composition d'ester de glycérol comprend au moins 90 % en poids de trivalérate de glycéryle, moins de 10 % en poids de divalérate de glycéryle et moins de 2,5 % en poids de monovalérate de glycéryle.

8. Composition d'ester de glycérol à utiliser selon l'une quelconque des revendications 1 à 7, dans laquelle la quantité d'acide valérique libre dans ladite composition d'ester de glycérol est inférieure à 1 % en poids.

9. Composition d'ester de glycérol à utiliser selon l'une quelconque des revendications 1 à 7, dans laquelle la quantité d'acide valérique libre dans ladite composition d'ester de glycérol est inférieure à 0,5 % en poids.

10. Composition d'ester de glycérol à utiliser selon l'une quelconque des revendications 1 à 9, dans laquelle ladite composition d'ester de glycérol est adsorbée sur un support, tel qu'un support de silice, permettant ainsi à ladite composition d'ester de glycérol d'être distribuée sous forme de produit sec.

11. Composition d'ester de glycérol à utiliser selon la revendication 10, dans laquelle ledit support comprend des particules de silice poreuses ayant une taille de particule moyenne de 20 à 70 µm.

12. Composition d'ester de glycérol à utiliser selon la revendication 10, dans laquelle ladite composition d'ester de glycérol est adsorbée sur des particules de silice dans un rapport pondéral de 50 à 80 % d'ester de glycérol et de 20 à 50 % de particules de silice.

13. Composition d'ester de glycérol à utiliser selon l'une quelconque des revendications 1 à 12, dans laquelle ladite composition d'ester de glycérol est introduite dans lesdits galloanserans dans une quantité comprise entre 0,05 à 1,5 % en poids de la ration alimentaire quotidienne des galloanserans.

14. Composition d'ester de glycérol à utiliser selon l'une quelconque des revendications 1 à 12, dans laquelle ladite composition d'ester de glycérol est utilisée pour nourrir lesdits galloanserans dans une quantité comprise entre 0,1 et 1,0 % en poids de la ration alimentaire quotidienne des galloanserans.
